(19) **Europäisches Patentamt**
**European Patent Office**
**Office européen des brevets**

(11) **EP 2 441 334 A1**

(12) **EUROPEAN PATENT APPLICATION**

(43) Date of publication:
**18.04.2012 Bulletin 2012/16**

(51) Int Cl.:
*A23P 1/16* (2006.01)   *A23L 1/00* (2006.01)
*A23L 3/015* (2006.01)

(21) Application number: **10187608.4**

(22) Date of filing: **14.10.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Unilever N.V.**
**3013 AL Rotterdam (NL)**

(72) Inventors:
• **Duggal, Charu**
  **Bangalore 560 066 (IN)**

• **Naik, Vijay Mukund**
  **Mumbai 400 063 (IN)**
• **Raut, Janhavi Sanjay**
  **Colworth sharnbrook**
  **Bedfordshir MK44 1LQ (GB)**
• **Stoyanov, Simeon Dobrev**
  **3133 AT Vlaardingen (NL)**

(74) Representative: **Hugot, Alain**
**Unilever NV**
**Patent Group**
**Olivier Van Noortlaan 120**
**3133 AT Vlaardingen (NL)**

(54) **A process for preparation of a foamed composition**

(57)    The invention relates to a process for preparation of a foamed composition and a foamed composition prepared thereby. According to the first aspect of the present invention there is provided a process to prepare a foamed composition comprising a liquid, gas bubbles dispersed therein and a foam stabilizer selected from hydrophobins wherein the process comprises a step of foaming by hydrodynamic cavitation. According to another aspect of the present invention there is provided a foamed composition prepared by the process of the invention.

EP 2 441 334 A1

SEQUENCE LISTING

<110>  Unilever

<120>  A process for preparation of a foamed composition

<130>  G2027EPff

<160>  2

<170>  PatentIn version 3.5

<210>  1
<211>  131
<212>  PRT
<213>  Artificial Sequence

<220>
<223>  unknown

<400>  1

```
Met Leu Lys Lys Ala Met Val Ala Ala Ala Ala Ala Ser Val Ile
1               5                   10                  15


Gly Met Ser Ala Ala Ala Ala Pro Gln Ala Leu Ala Ile Gly Asp Asp
            20                  25                  30


Asn Gly Pro Ala Val Ala Asn Gly Asn Gly Ala Glu Ser Ala Phe Gly
            35                  40                  45


Asn Ser Ala Thr Lys Gly Asp Met Ser Pro Gln Leu Ser Leu Val Glu
            50                  55                  60


Gly Thr Leu Asn Lys Pro Cys Leu Gly Val Glu Asp Val Asn Val Ala
65                  70                  75                  80


Val Ile Asn Leu Val Pro Ile Gln Asp Ile Asn Val Leu Ala Asp Asp
                85                  90                  95


Leu Asn Gln Gln Cys Ala Asp Asn Ser Thr Gln Ala Lys Arg Asp Gly
                100                 105                 110


Ala Leu Ser His Val Leu Glu Asp Leu Ser Val Leu Ser Ala Asn Gly
            115                 120                 125


Glu Gly Arg
    130
```

<210>  2
<211>  133
<212>  PRT
<213>  Artificial Sequence

<220>
<223>  unknown

<400>  2

Met Ile Lys Lys Val Val Ala Tyr Ala Ala Ile Ala Ala Ser Val Met
1                   5                   10                  15

Gly Ala Ser Ala Ala Ala Ala Pro Gln Ala Met Ala Ile Gly Asp Asp
              20                  25                  30

Ser Gly Pro Val Ser Ala Asn Gly Asn Gly Ala Ser Gln Tyr Phe Gly
          35                  40                  45

Asn Ser Met Thr Thr Gly Asn Met Ser Pro Gln Met Ala Leu Ile Gln
      50                  55                  60

Gly Ser Phe Asn Lys Pro Cys Ile Ala Val Ser Asp Ile Pro Val Ser
65                  70                  75                  80

Val Ile Gly Leu Val Pro Ile Gln Asp Leu Asn Val Leu Gly Asp Asp
              85                  90                  95

Met Asn Gln Gln Cys Ala Glu Asn Ser Thr Gln Ala Lys Arg Asp Gly
              100                 105                 110

Ala Leu Ala His Leu Leu Glu Asp Val Ser Ile Leu Ser Ser Asn Gly
              115                 120                 125

Glu Gly Gly Lys Gly
    130

**Description**

**TECHNICAL FIELD**

The invention relates to a process for preparation of a foamed composition and a foamed composition prepared thereby. The invention more particularly relates to food compositions comprising gas bubbles that remain entrained for long periods of time in the compositions and have a unique shape and size.

**BACKGROUND AND PRIOR ART**

There are many compositions that consumers use in which gas bubbles have been incorporated for providing many benefits which appeal to the consumer. Such products include cosmetics and foods. Foamed compositions find visual and sensory appeal in cosmetic compositions e.g. skin creams, shaving creams, hair products, liquid soaps etc. In the area of foods, gas bubbles have been incorporated for giving not just visual or sensory appeal to the products but for providing functional benefits. Many consumers prefer to eat large quantity of food but are unable to do so since they are weight conscious and want to reduce their intake of food that is high in carbohydrate, fat and other high calorie items. With such foods, foamed compositions have been prepared where the fat and carbohydrate contents have been reduced and replaced with gas bubbles. Such foamed food products give the consumer a feeling of satiety while ensuring minimization of calorie intake. The inclusion of gas e.g. air in the form of foams also provides for a unique mouth feel in many food products which are liked by many consumers. Further, the inclusion of foams in food products gives the product a different visual appeal, often quite attractive. Examples of such foamed food composition include whipped cream, butter, and cheese which are consumed at room temperature; ice cream which is consumed at low temperature; and beverages like chocolate, coffee, milkshakes etc which may be consumed either hot or cold.

One of the problems with foamed compositions is that the foams tend to be unstable when the compositions containing them are stored over long periods of time e.g. over days and months. By the term unstable, is meant that the foam size tends to increase over time and all parts of a packaged composition, often, do not have the same amount of foam present in it. One example that many consumers have experienced is that while the top of the package on early use tends to be high in foam, the bottom of the package is flat and does not contain high amount of foam, as desired. Ways in which this stability problem have been tackled include incorporation of foam stabilizers in the foamed compositions. Commonly used foam stabilizers include emulsifiers which are surface active agents, proteins, and thickeners like gums e.g. alginates, gelatin, guar gums, pectins etc. These agents have had limited ability to stabilize the compositions and there are disadvantages in each one of them. While gums as thickeners have been the most common and successfully used foamed stabilizers, they have the inherent disadvantage of thickening the products thus making them unsuitable for use in products where high fluidity is desirable e.g. beverages. While, emulsifiers have less disadvantage in terms of reducing the fluidity of the product, they are not as good a foam stabilizer as gums. Thus, there has been a need in the art to develop materials that provide enhanced stabilization of foams in foamed compositions. The present inventors have been working on this for the last several years and a few prior patent applications from this group have been published in the last few years.

WO2008/046698 (Unilever) is a patent publication which discloses food composition comprising gas bubbles and processes for preparing them. This patent application discloses aerated food product in the form of a stable foam comprising 5 - 80 vol% gas bubbles, 15 - 90% water, and 0.001 to 10 wt% fibres assembled with surface active particles at the air water interface. Thus, this publication and many other subsequent patent applications from the present applicants have been significant improvements in producing foamed food compositions over the then prior art, the present group of researchers have been continuously working to improve them even further.

In developing the present invention, the inventors worked extensively on many process variations and finally hit upon a process viz. hydrodynamic cavitation in preparing such foamed compositions, which have not been extensively used in food processing. Surprisingly, when this process was used along with a specific foam stabliser viz. hydrophobins, the foamed compositions thus prepared provided certain benefits in both the size and shape of the gas bubbles with concomitant high stability of the foamed composition.

Hydrodynamic cavitation has been known and used in the chemical processing industry e.g. US5085371 (Shop Vac Corp, 1992) discloses a nozzle assembly for producing a mousse-like mixture of air and a foamable liquid which includes a housing defining a passageway and a venturi located downstream of a mixing chamber also located in the passageway.

However, the above publications does not disclose that a unique combination of a specific class of foam stabilizer viz. hydrophobins which in combination with the selective process of generation of gas bubbles viz. hydrodynamic cavitation can be used for preparing a foamed composition where the foams have distinct bubble size/ shape and are also highly stable.

It is an object of the present invention to provide for a process to prepare a foamed composition where the foam principally comprises of gas bubbles having diameter in the range of 1 to 20 micrometer.

It is yet another object of the present invention to provide for a foamed composition having gas bubbles with diameter in the range of 1 to 20 micrometer where the foam is highly stable.

## SUMMARY OF THE INVENTION

According to the first aspect of the present invention there is provided a process to prepare a foamed composition comprising a liquid, gas bubbles dispersed therein and a foam stabilizer selected from hydrophobins wherein the process comprises a step of foaming by hydrodynamic cavitation, and wherein said hydrodynamic cavitation is created using a converging-diverging nozzle, and the process comprises the steps of (i) preparing a solution/dispersion comprising said liquid and said foam stabilizer and (ii) passing said solution/ dispersion through said converging-diverging nozzle using a pump, and wherein the Reynolds number at the throat of the converging diverging nozzle is at least 3500.

It is particularly preferred that the hydrophobin is a class II hydrophobhin.

According to another aspect of the present invention there is provided a foamed composition prepared by the process of the invention.

## BRIEF DESCRIPTION OF THE FIGURES

Figure 1: depicts a schematic of a device for preparing the foamed composition of the invention.
Figure 2: Stability of foams of Example 2, Figure 2(a) shows the microscopy picture of fresh foam and Figure 2(b) the microscopy picture of aged foam after 30 days.
Figure 3: Stability of foams of Example 3., Figures 3(a) to 3(c) shows the foam height for the fresh and aged foams of corresponding Examples 3(a) to 3(c)
Figure 4: Microscopy pictures of the bubbles of Example 4, Figures 4(a): obtained using the hydrodynamic cavitation Figure 4(b) obtained using the conventional process
Figure 5: Foam of Example 5 redispersed: Figure 5(a): HFB Foam redispersed in DI water, Figure 5(b): HFB Foam redispersed in tomato sauce, Figure 5(c): HFB Foam redispersed in Mayonaise. (Scale Bar = 100 microns)
Figure 6: Foam of Example 6 redispersed.: Figure 6(a): HFB Foam redispersed in Body Lotion, Figure 6(b): HFB Foam redispersed in Hair Conditioning gel (Scale Bar = 100 microns)

## DETAILED DESCRIPTION OF THE INVENTION

The invention provides for a process to prepare a foamed composition. The composition comprises a liquid, gas bubbles dispersed therein and a foam stabilizer. The liquid may be water, oil, solvents, gels, polymeric liquids, emulsions, suspensions or mixtures there of. The liquid is preferably water, solvent or oil, most preferably water. The gas which is present in the form of bubbles in the liquid is air, oxygen, nitrogen, nitrous oxide, helium, or carbon dioxide. The gas could also be any other gas which is relatively inert and have no adverse reaction when contacted with the outside surface of the human body or consumed internally. The more preferred gas present in the form of bubbles in the foamed composition is air or nitrogen, most preferably air. Thus, when air is the gas forming the foam, the composition is called aerated composition. Alternately, the gas may be introduced into the liquid in the dissolved form and could be replenished over cycles. The foam stabiliser is selected from a class of compounds known as hydrophobins. These foam stabilizers provide foam stability for more than a week of storage.

Hydrophobins are a well-defined class of proteins (Wessels, 1997, Adv. Microb. Physio. 38: 1-45; Wosten, 2001, Annu Rev. Microbiol. 55: 625-646) capable of self-assembly at a hydrophobic/hydrophilic interface, and having a conserved sequence:

$$\text{Xn-C-X5-9-C-C-X11-39-C-X8-23-C-X5-9-C-C-X6-18-C-Xm}\quad\text{(SEQ ID No. 1)}$$

where X represents any amino acid, and n and m independently represent an integer. Typically, a hydrophobin has a length of up to 125 amino acids. The cysteine residues (C) in the conserved sequence are part of disulphide bridges. In the context of the present invention, the term hydrophobin has a wider meaning to include functionally equivalent proteins still displaying the characteristic of self-assembly at a hydrophobic-hydrophilic interface resulting in a protein film, such as proteins comprising the sequence:

Xn-C-X1-50-C-X0-5-C-X1-100-C-X1-100-C-X1-50-C-X0-5-C-X1-50-C-Xm (SEQ ID No. 2)

or parts thereof still displaying the characteristic of self-assembly at a hydrophobic-hydrophilic interface resulting in a protein film. In accordance with the definition of the present invention, self-assembly can be detected by adsorbing the protein to Teflon and using Circular Dichroism to establish the presence of a secondary structure (in general, $\alpha$-helix) (De Vocht et al., 1998, Biophys. J. 74: 2059-68).

The formation of a film can be established by incubating a Teflon sheet in the protein solution followed by at least three washes with water or buffer (Wosten et al., 1994, Embo. J. 13: 5848-54). The protein film can be visualised by any suitable method, such as labeling with a fluorescent marker or by the use of fluorescent antibodies, as is well established in the art. m and n typically have values ranging from 0 to 2000, but more usually m and n in total are less than 100 or 200. The definition of hydrophobin in the context of the present invention includes fusion proteins of a hydrophobin and another polypeptide as well as conjugates of hydrophobin and other molecules such as polysaccharides.

Hydrophobins identified to date are generally classed as either class I or class II. Both types have been identified in fungi as secreted proteins that self-assemble at hydrophobilic interfaces into amphipathic films. Assemblages of class I hydrophobins are relatively insoluble whereas those of class II hydrophobins readily dissolve in a variety of solvents.

Hydrophobin-like proteins have also been identified in filamentous bacteria, such as Actinomycete and Steptomyces sp. (WO01/74864) These bacterial proteins, by contrast to fungal hydrophobins, form only up to one disulphide bridge since they have only two cysteine residues. Such proteins are an example of functional equivalents to hydrophobins having the consensus sequences shown in SEQ ID Nos. 1 and 2, and are within the scope of the present invention.

The hydrophobins can be obtained by extraction from native sources, such as filamentous fungi, by any suitable process. For example, hydrophobins can be obtained by culturing filamentous fungi that secrete the hydrophobin into the growth medium or by extraction from fungal mycelia with 60% ethanol. It is particularly preferred to isolate hydrophobins from host organisms that naturally secrete hydrophobins. Preferred hosts are hyphomycetes (e.g. Trichoderma), basidiomycetes and ascomycetes. Particularly preferred hosts are food grade organisms, such as Cryphonectria parasitica which secretes a hydrophobin termed cryparin (MacCabe and Van Alfen, 1999, App. Environ. Microbiol 65: 5431-5435).

Alternatively, hydrophobins can be obtained by the use of recombinant technology. For example host cells, typically micro-organisms, may be modified to express hydrophobins and the hydrophobins can then be isolated and used in accordance with the present invention. Techniques for introducing nucleic acid constructs encoding hydrophobins into host cells are well known in the art. More than 34 genes coding for hydrophobins have been cloned, from over 16 fungal species (see for example WO96/41882 which gives the sequence of hydrophobins identified in Agaricus bisporus; and Wosten, 2001, Annu Rev. Microbiol. 55: 625-646). Recombinant technology can also be used to modify hydrophobin sequences or synthesise novel hydrophobins having desired/improved properties.

Typically, an appropriate host cell or organism is transformed by a nucleic acid construct that encodes the desired hydrophobin. The nucleotide sequence coding for the polypeptide can be inserted into a suitable expression vector encoding the necessary elements for transcription and translation and in such a manner that they will be expressed under appropriate conditions (e.g. in proper orientation and correct reading frame and with appropriate targeting and expression sequences). The methods required to construct these expression vectors are well known to those skilled in the art.

A number of expression systems may be used to express the polypeptide coding sequence. These include, but are not limited to, bacteria, fungi (including yeast), insect cell systems, plant cell culture systems and plants all transformed with the appropriate expression vectors. Preferred hosts are those that are considered food grade - 'generally regarded as safe' (GRAS).

Suitable fungal species, include yeasts such as (but not limited to) those of the genera Saccharomyces, Kluyveromyces, Pichia, Hansenula, Candida, Schizo saccharomyces and the like, and filamentous species such as (but not limited to) those of the genera Aspergillus, Trichoderma, Mucor, Neurospora, Fusarium and the like.

The sequences encoding the hydrophobins are preferably at least 80% identical at the amino acid level to a hydrophobin identified in nature, more preferably at least 95% or 100% identical. However, persons skilled in the art may make conservative substitutions or other amino acid changes that do not reduce the biological activity of the hydrophobin. For the purpose of the invention these hydrophobins possessing this high level of identity to a hydrophobin that naturally occurs are also embraced within the term "hydrophobins".

Hydrophobins can be purified from culture media or cellular extracts by, for example, the procedure described in WO01/57076 which involves adsorbing the hydrophobin present in a hydrophobin-containing solution to surface and then contacting the surface with a surfactant, such as Tween 20, to elute the hydrophobin from the surface. See also Collen et al., 2002, Biochim Biophys Acta. 1569: 139-50; Calonje et al., 2002, Can. J. Microbiol. 48: 1030-4; Askolin et al., 2001, Appl Microbiol Biotechnol. 57: 124-30; and De Vries et al., 1999, Eur J Biochem. 262: 377-85.

It is preferred that the hydrophobins for use in the present invention are Class II hydrophobins. Class II hydrophobins are usually soluble in a variety of solvents including water. It is further preferred that the hydrophobins are in isolated form. This isolated form is typically at least partially purified, such that at least 10% pure, based on weight of solids. By "isolated form" is meant that the hydrophobin is not added as part of a naturally-occuring organism, such as a mushroom, which naturally expresses hydrophobins. Instead, the hydrophobin will typically either have been extracted from a naturally-occuring source or obtained by recombinant expression in a host organism.

The essential step of producing the foam in the composition is hydrodynamic cavitation. Cavitation is the formation and subsequent implosion of bubbles or cavities in a liquid. When the local pressure in a liquid becomes lower than the vapour pressure of the liquid (at that temperature) or when the local pressure turns negative (signifying tension rather than compression), small bubbles or cavities form. These cavities may be entirely newly formed when the negative pressures are large enough to overcome the attractive van der Waals forces between molecules. Theoretically, one can show that, for water, this would occur if the negative pressure is about 4000 N/m$^2$. Alternatively (and realistically), small pre-existing bubbles may act as nuclei, leading to bubble expansion. On reaching high pressure regions, these bubbles can collapse catastrophically, leading to generation of high pressures, temperatures and velocities. Cavitation is usually of two types - hydrodynamic cavitation, produced by pressure variations in a flowing liquid due to the geometry of the system, and the more familiar acoustic cavitation, produced by sound waves in a liquid. Hydrodynamic cavitation in flowing systems can be created through the use of convergent-divergent nozzles geometries where the flowrate is adjusted such that the velocity head increases significantly thereby reducing the pressure head below the vapour pressure of the fluid.

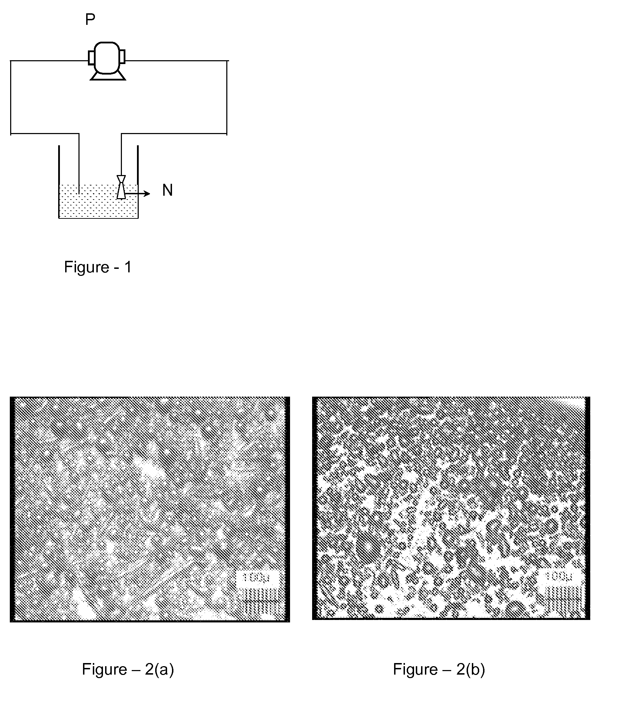

The hydrodynamic cavitation, in the present invention is preferably created using a converging-diverging nozzle. Thus, a suitable way of preparing the foamed composition of the invention comprises taking the desired liquid in a vessel. The foam stabilizer is then dissolved/ dispersed in the liquid through appropriate means. If the foam stabilizer is insoluble in the liquid, it may be dispersed in the liquid through use of a second liquid in which the foam stabilizer is soluble. The second liquid may be an organic solvent. Suitable second liquids are ethanol or acetone. Once the foam stabliser, the desired liquid and the second liquid are well mixed, the second liquid may be suitably removed from the mixture by any known method. If the second liquid is a volatile organic solvent, it may be removed by evaporation under vacuum. The mixture of the desired liquid and the foam stabilizer is then taken in a vessel in which the converging-diverging nozzle is immersed. The mixture is then recirculated through the converging-diverging nozzle through a suitable pump. Suitable pumps are positive displacement pumps like peristaltic or gear pumps which can build suitable head pressure to provide the required flowrates. Centrifugal pumps can also be used for the process. The mixture may be circulated for a sufficient period of time to simulate a single pass through the pump or may be long enough to encompass multiple passes. Alternatively dissolved gases and/or small pre-existing bubbles can act as nuclei, leading to bubble expansion. The bubbles which thereby constitute both the vapour and the gases once formed get stabilized by the foam stabilizer present in the liquid. Figure 1 depicts a schematic of a device for preparing the foamed composition of the invention. In Figure 1, N represents a converging-diverging nozzle and P a pump.

The Reynolds number at the throat of the converging diverging nozzle is at least 3500, preferably at least 3500, more preferably at least 10000. It is particularly preferred that the Reynolds number at the throat of the converging diverging nozzle is greater than 12500, or even more preferably greater than 15000. The term throat as used herein means the region between the converging and the diverging sections where the cross-sectional area for the flow is the smallest.

**The Reynolds number at the throat is defined as**

$$Re = D \rho U / \mu$$

Where D is hydraulic diameter at the throat, defined as 4A/P where A is the cross-sectional area at the throat and P is the wetted perimeter.

U is the liquid velocity at the throat
$\mu$ is the viscosity of the liquid
$\rho$ is the density of the liquid

Thus, a preferred process comprises the steps of (i) preparing a solution/dispersion comprising the liquid and the foam stabilizer and (ii) passing said solution/ dispersion through said converging-diverging nozzle using a pump. The solution/ dispersion is preferably passed through the converging-diverging nozzle at a velocity greater than 10 m/s, more preferably greater than 15 m/s. The driving pressure is determined by the wall friction and this is preferably low enough to permit a sufficiently low pressure at the throat region for cavitation to occur. The low pressure requirement at the throat can be increased through increase in temperature or use of volatile solvents, dissolved gases or seeding agents. The wall friction can be reduced through use of appropriate material of construction, wall geometries as well as drag reducing agents. The inlet/driving pressure (gauge) at which the solution/ dispersion is passed through the converging-diverging nozzle is preferably in the range of $10^4$ to $10^7$ N/m$^2$, more preferably in the range of $10^4$ to $10^6$ N/m$^2$.

It is preferred that external gas phase introduced in the liquid is less than 10% by volume of the liquid phase. The term "external gas phase" as used herein excludes the bubbles formed due to cavitation. It is further preferred that external gas phase introduced in the composition is less that 5% by volume of the liquid phase. It is particularly preferred that no external gas phase is introduced during the process.

Preferably, the converging section does not have any inlet for external gas such that no external gas is sucked in by the Venturi effect during step (ii).

The process of the invention may optionally comprise additional steps subsequent to the essential steps of the process of the present invention. Optional steps may be cooling, freezing, gellation or structuring.

According to another aspect of the present invention there is provided a foamed composition prepared by a process of the invention. The composition prepared by the process of the present invention may have gas bubbles in a substantially spherical or elongate shape, elongate shapes being more preferred. Foamed composition prepared by the process of the present invention preferably has the diameter of the gas bubbles less than 20 micrometer, preferably in the range of 1 to 20 micrometer. In the present invention, the diameter of an ellipsoidal (elongated) bubble is the minor diameter.

The foamed composition of the invention may comprise the foam stabilizer, the liquid and the gas bubbles over a large range of concentration depending upon the use of the composition. In general, it is preferred that the foam stabilizer is 0.01 to 20 %, more preferably 1 to 5% by weight of the composition. The liquid is preferably present in 10 to 95%, more preferably 10 to 50% by volume of the composition. The gas bubbles are preferably present in 5 to 90 %, more preferably 50 to 80% by volume of the composition.

The foamed composition has applications over a wide range of consumer products, most preferred being food products or cosmetic products e.g. for application on skin, hair or other external surfaces of the body.

The present invention is especially suitable for preparing food product. Food product that may be prepared include whipped cream, cheese, butter or fat, ice-cream, soups, sauces, yoghurts, custards, spreads, chocolates or beverage compositions comprising coffee, tea, milkshakes or buttermilk.

When the product is a frozen product, it is prepared by the process of the invention to make a foamed composition that comprises liquid, gas bubbles dispersed therein and the foam stabilizer. The foamed composition may then be cooled or frozen to the desired temperature to prepare the frozen product which may be a gel, a soft or hard solid. In the final frozen product, it is possible that the liquid originally subjected to the process of the invention is in the solid state e.g. in the form of ice crystals.

The composition of the invention has a %overrun which is preferably is in the range of 2 to 200%, more preferably in the range of 10 to 100%. The term "overrun" refers to the ratio of the gas volume in the foam and the volume of the liquid involved in its formation expressed as a percentage value. Thus,

$$\%Overrun = [(V_{total} - V_{liquid})/V_{liquid}] \times 100\%$$

where $V_{liquid}$ = initial volume of the liquid and
$V_{total}$ = final volume of the liquid-gas mixture after aeration. The %overrun thus refers to the extent of gas incorporated through the process.

The invention will now be illustrated with the help of the following non-limiting examples.

**EXAMPLES**

Example 1: Process for creation of foamed composition as per the invention using Hydrophobin as foam stabiliser and using step of Hydrodynamic Cavitation

Example 1-2: Effect of Flowrate on the extent of overrun achieved:

0.1% solution of Hydrophobin (HFBII - obtained from VTT Biotechnology, Finland) was prepared. 100 ml of the solution was circulated through a cavitation nozzle using the process scheme as shown in Figure 1 at a flowrate of 0.25 l/min (example 1a). The nozzle outlet was immersed entirely in the fluid mass to avoid air entrainment through surface turbulence. The solution was circulated for 10 minutes. The extent of overrun obtained was noted. The same experiment was repeated for 100 ml aliquots of the HFB solutions at different flowrates as shown in table 1 below. The throat velocity and corresponding throat Reynolds number was calculated and shown in the table. The viscosity of the dispersion was measured as - 1.8 cp using a Brookefields viscometer. The density of the dispersion was - 1gm/cc.

Table 1 - % overrun as a function of Reynolds number.

| Example | Flowrate | D(throat) | V (throat) | Re(throat) | Overrun (after 10 min) |
|---------|----------|-----------|-----------|-----------|------------------------|
| 1 | 0.25 litres/min | 1 mm | 5.3 m/s | 2945 | 0 |
| 2 | 1.5 litres/min | 1 mm | 31.8 m/s | 17670 | 50 |

It can be seen that for Example 1 with the Reynolds number at throat greater than 3500 (within the scope of the invention), the cavitation resulted in an overrun of 50%, as compared to the case of Example 2 with the Reynolds number at throat less than 3500 (outside the scope of the invention), the cavitation resulted in no overrun. For the Example 2, the resultant foam was stable up to 30 days. Figure 2(a) shows the microscopy picture of fresh foam and Figure 2(b) the microscopy picture of aged foam after 30 days. The foam was stored at -5°C. Thus, foamed compositions can be prepared by the process of the invention using hydrophobin as the foam stabiliser.

Example 3: Stability of foam created using Hydrodynamic Cavitation using other stabilizers.

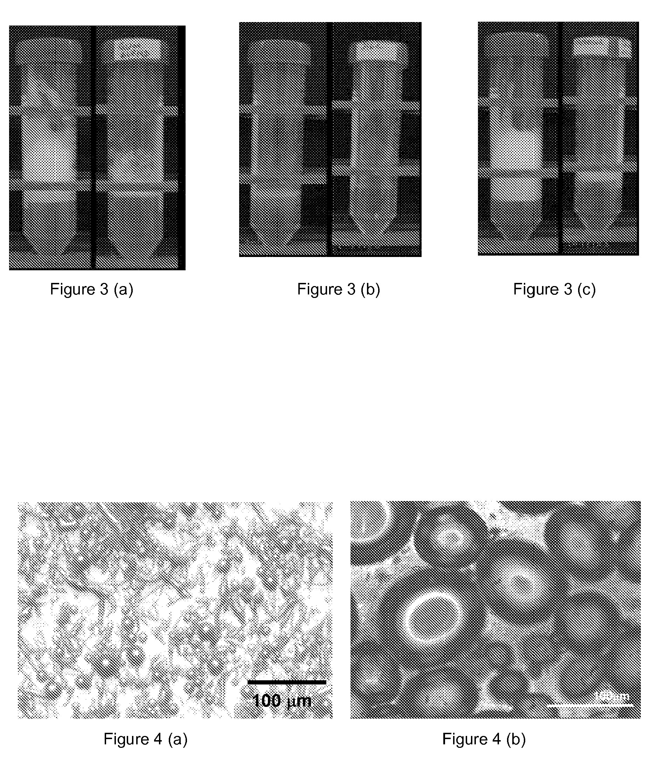

Solutions/dispersions of various foam stabilizers used in food products as listed in Table 2 below were used for creating foams using hydrodynamic cavitation using the process scheme as shown in Figure -1. The throat diameter was 1 mm and flowrate was 1 L/min. The Reynolds number was about 10000 in all the caaes. The solutions/dispersions were circulated for 10 minutes. The foams were collected in calibrated tubes and the foam height observed over a period of time. Figures 3(a) to 3(c) shows the foam height for the fresh and aged foams (stored for 15 days) prepared using the different solutions/dispersions.

Table 2

| Example | Foam Stabiliser | Within the invention? | Concentration, Wt% | Figure depicting Foam |
|---------|-----------------|----------------------|--------------------|-----------------------|
| 3(a) | Gum blend* | No | 0.2% | 3(a) |
| 3(b) | Hydroxyethyl cellulose [09368 from Fluka, Biochemica | No | 0.5% | 3(b) |
| 3(c) | Methocel Food Grade Hydroxy Propyl Cellulose [E464 from the Dow Chemical Co. | No | 0.5% | 3(c) |
| * Gum blend here is a blend of glycerol mono stearate (60%), Locust bean gum (24%), Guar Gum (12%), Carrageenan (4%). | | | | |

The Figures 3(a) to 3(c) indicate that use of foam stabiliser outside the invention provide for poor foam stability.

Example 4: Bubble size obtained in foamed compositions with step of hydrodynamic cavitation as compared to conventional mixing step

Experiments were conducted to determine the characteristics of foamed compositions using the step of hydrodynamic cavitation in comparison to conventional mixing step. These experiments were conducted as follows:

In the process comprising the step of hydrodynamic cavitation, dispersions of the foam stabiliser were circulated through a cavitation nozzle using the process scheme as shown in Figure 1. In the conventional process the same dispersion was whipped using a Kenwood Kitchen mixer at full power for 2 minutes. The experiments are summarised in Table 2 and the results are depicted in Figures 4(a) and 4(b) in terms of microscopy pictures of the bubbles obtained using the hydrodynamic cavitation and the conventional process, respectively

Table 2

| Example | Process step | Foam Stabiliser | Concentration of foam stabiliser | Result in Figure |
|---------|--------------|-----------------|----------------------------------|------------------|
| 4(a) | Hydrodynamic cavitation | Hydrophobin (HFBII) | 0.1% | 4(a) |

(continued)

| Example | Process step | Foam Stabiliser | Concentration of foam stabiliser | Result in Figure |
|---------|--------------|-----------------|----------------------------------|------------------|
| 4(b) | Conventional process | Hydrophobin (HFBII) | 0.1% | 4(b) |

The Figures 4(a) and 4(b) indicate that process as per the invention (Figure 4(a)) provides for foamed composition with much smaller bubble sizes as compared to conventional process.

Example 5:

Introduction of Preformed HFB Foam into Food Products:

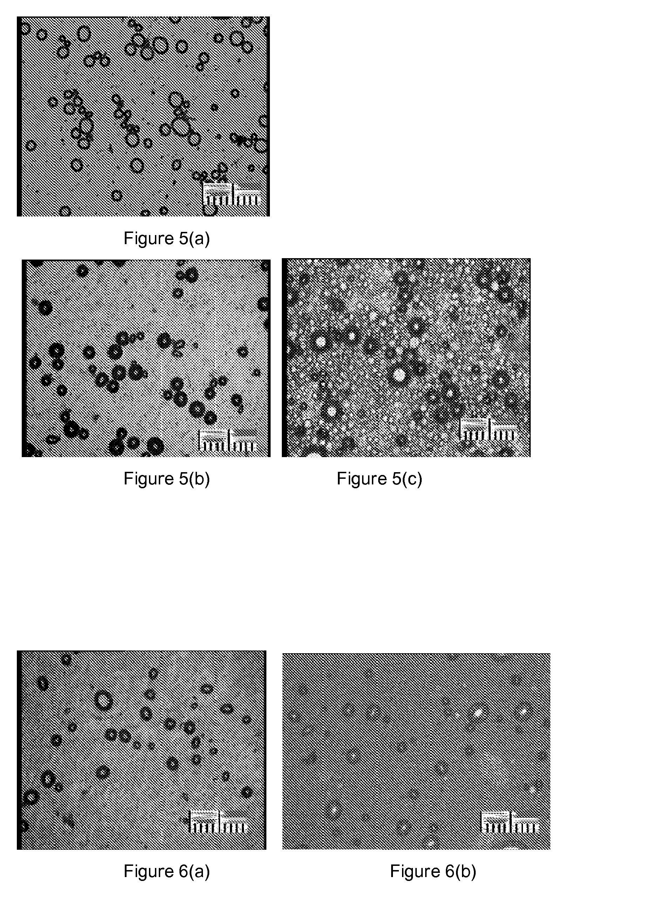

HFB Foam was prepared as per the process described in Example1 (b). The stored foam was redispersed into Foods products like tomato sauce (Maggier® - Nestle) and Mayonaise (Classic Creamy ® - Unilever) by stirring in the foam using a spatula. The ratio of foam to product was 20% by volume. It should be noted that since the foam was not completely dry, some amount of liquid gets entrained with the foam. The aerated products were observed under the microscope. Figures 5(a), 5(b) and 5(c) show micrographs of the HFB foam redispersed in deionised water, foam redispersed in tomato sauce and foam redispersed in mayonnaise respectively. As seen in the figures, the bubble size is conserved in the high viscosity complex products as well.

Example 6:

Introduction of Preformed HFB Foam into Personal Care Products:

HFB Foam was prepared as per the process described in Example 1(b). The stored foam was redispersed into Personal care products- body lotion (Vaselin®- Unilever and hair conditioning gel (Dove® - Unilever) by stirring in the foam using a spatula The ratio of foam to product was 20% by volume. It should be noted that since the foam was not completely dry, some amount of liquid gets entrained with the foam. The aerated product was observed under the microscope. Figures 6(a), and 6(b) show micrographs of the HFB foam redispersed in body lotion and in hair gel respectively. As seen in the figures, the bubble size is conserved in the high viscosity complex products as well.

**Claims**

1.  A process to prepare a foamed composition comprising a liquid, gas bubbles dispersed therein and a foam stabilizer selected from hydrophobins wherein the process comprises a step of foaming by hydrodynamic cavitation, and said hydrodynamic cavitation is created using a converging-diverging nozzle, and the process comprises the steps of (i) preparing a solution/dispersion comprising said liquid and said foam stabilizer and (ii) passing said solution/ dispersion through said converging-diverging nozzle using a pump, and wherein the Reynolds number at the throat of the converging diverging nozzle is at least 3500.

2.  A process as claimed in claim 1 wherein said converging section does not have any inlet for external gas such that no external gas is sucked in by the Venturi effect during step (ii).

3.  A process as claimed in claim 1 or 2 wherein said hydrophobin is a class II hydrophobin.

4.  A process as claimed in any one of the preceding claims 2 wherein said hydrophobin is in isolated form.

5.  A process as claimed in any one of the preceding claims wherein said solution/ dispersion is passed through said converging-diverging nozzle at a maximum velocity greater than 10 m/s.

6.  A process as claimed in any one of the preceding claims wherein said solution/dispersion is passed though said converging-diverging nozzle at a pressure in the range of $10^4$ to $10^7$ N/m$^2$.

7. A process as claimed in any one of the preceding claims wherein said liquid is water, solvent, or oil.

8. A process as claimed in any one of the preceding claims wherein said gas bubbles comprise air, oxygen, nitrogen, nitrous oxide, helium, or carbon dioxide.

9. A process as claimed in any one of the preceding claims wherein said foam stabilizer is present in 0.01 to 20 % by weight of the composition.

10. A foamed composition prepared by a process as claimed in any one of the preceding claims.

11. A composition as claimed in claim 10 for application in foods or cosmetics.

12. A composition as claimed in claim 11 wherein said food is whipped cream, cheese, butter or fat, ice-cream, soup, sauce, yoghurt, custard, spread, chocolate or a beverage composition comprising coffee, tea, milkshakes or buttermilk.

Figure - 1

Figure – 2(a)

Figure – 2(b)

Figure 3 (a)          Figure 3 (b)          Figure 3 (c)

Figure 4 (a)                    Figure 4 (b)

Figure 5(a)

Figure 5(b)

Figure 5(c)

Figure 6(a)

Figure 6(b)

Europäisches
Patentamt

European
Patent Office

Office européen
des brevets

# EUROPEAN SEARCH REPORT

**Application Number**

EP 10 18 7608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TCHUENBOU-MAGAIA F L ET AL: "Hydrophobins stabilised air-filled emulsions for the food industry", FOOD HYDROCOLLOIDS, ELSEVIER, vol. 23, no. 7, 1 October 2009 (2009-10-01), pages 1877-1885, XP026145883, ISSN: 0268-005X, DOI: DOI:10.1016/J.FOODHYD.2009.03.005 [retrieved on 2009-03-24] | 10,11 | INV. A23P1/16 A23L1/00 A23L3/015 |
| Y | * abstract * <br> * Page 1878, right column, section 2.3.1 * <br> * Page 1879, right column, the paragraph starting with "The air cells in the AFE ..." * <br> * Page 1881, right column, first paragraph * <br> * Page 1884, "Conclusion" * | 1-9 | |
| X | EP 1 938 697 A1 (UNILEVER PLC [GB]; UNILEVER NV [NL]) 2 July 2008 (2008-07-02) | 10-12 | |
| Y | * claims 1-14; example 5 * | 1-9 | **TECHNICAL FIELDS SEARCHED (IPC)** |
| X | COX A R ET AL: "Exceptional stability of food foams using class II hydrophobin HFBII", FOOD HYDROCOLLOIDS, ELSEVIER, vol. 23, no. 2, 1 March 2009 (2009-03-01), pages 366-376, XP026128626, ISSN: 0268-005X, DOI: DOI:10.1016/J.FOODHYD.2008.03.001 [retrieved on 2008-03-07] | 10-12 | A23C A23G A23L A23P A47J A61K |
| Y | * Page 367, right column, last paragraph before section 2. "Materials and methods" * <br> * Page 368, right column, Table 1 * <br> * Page 370, left column, section 2.6 * <br> * Page 374, left column, section 3.5 * | 1-9 | |

-/--

The present search report has been drawn up for all claims

| Place of search | Date of completion of the search | Examiner |
|---|---|---|
| Munich | 30 May 2011 | Götz, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EUROPEAN SEARCH REPORT

Application Number

EP 10 18 7608

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2010/104705 A1 (GORDON ROMAN [US] ET AL) 29 April 2010 (2010-04-29)<br>* figure 2 *<br>* paragraphs [0023] - [0024] *<br>* paragraphs [0034] - [0035] *<br>* paragraphs [0074] - [0076] *<br>* claim 1 *<br>----- | 1-9 | |

TECHNICAL FIELDS
SEARCHED        (IPC)

The present search report has been drawn up for all claims

| Place of search | Date of completion of the search | Examiner |
|---|---|---|
| Munich | 30 May 2011 | Götz, Michael |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 18 7608

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2011

| Patent document cited in search report | | Publication date | Patent family member(s) | Publication date |
|---|---|---|---|---|
| EP 1938697 | A1 | 02-07-2008 | NONE | |
| US 2010104705 | A1 | 29-04-2010 | WO 2010062600 A1 | 03-06-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008046698 A
- US 5085371 A
- WO 0174864 A
- WO 9641882 A
- WO 0157076 A

### Non-patent literature cited in the description

- **WESSELS.** *Adv. Microb. Physio.,* 1997, vol. 38, 1-45
- **WOSTEN.** *Annu Rev. Microbiol.,* 2001, vol. 55, 625-646
- **DE VOCHT et al.** *Biophys. J.,* 1998, vol. 74, 2059-68
- **WOSTEN et al.** *Embo. J.,* 1994, vol. 13, 5848-54
- **MACCABE ; VAN ALFEN.** *App. Environ. Microbiol,* 1999, vol. 65, 5431-5435
- **COLLEN et al.** *Biochim Biophys Acta,* 2002, vol. 1569, 139-50
- **CALONJE et al.** *Can. J. Microbiol.,* 2002, vol. 48, 1030-4
- **ASKOLIN et al.** *Appl Microbiol Biotechnol.,* 2001, vol. 57, 124-30
- **DE VRIES et al.** *Eur J Biochem.,* 1999, vol. 262, 377-85